# EUROPEAN PATENT SPECIFICATION

(11) **EP 3 475 311 B1**
(45) Date of publication and mention of the grant of the patent: **13.05.2020**
(21) Application number: 17734661.6
(22) Date of filing: 19.06.2017
(51) Int. Cl.: C08B 37/02, A61P 13/08

(54) **MODIFIED DEXTRAN CONJUGATES COMPRISING A LYSINE-UREA-GLUTAMATE PHARMACOPHORE**
MODIFIZIERTE DEXTRANKONJUGATE MIT EINEM LYSIN-HARNSTOFF-GLUTAMAT-PHARMAKOPHOR
CONJUGUÉS DE DEXTRANE MODIFIÉS COMPRENANT UN PHARMACOPHORE LYSINE-URÉE-GLUTAMATE

(30) Priority: 22.06.2016 FI 20165515
(43) Date of publication of application: 01.05.2019
(73) Proprietor: Dextech Medical AB, 75106 Uppsala (SE)
(72) Inventor: HOLMBERG, Anders R, 75106 Uppsala (SE); NILSSON, Sten, 75322 Uppsala (SE)
(74) Representative: Boco IP Oy Ab
(86) International application number: PCT/EP2017/064929
(87) International publication number: WO 2017/220488

(56) References cited:
- EP-A1- 3 011 976
- WO-A1-2009/124580
- WO-A1-2014/127365
- WO-A2-2008/058192
- WO-A2-2008/107471
- WO-A2-2015/058151

## Description

### Field of the Invention

The present invention is related to modified dextran conjugates, such as guanidine-dextran conjugates, comprising a glutamate-urea-lysine pharmacophore, and a method for preparation of said conjugates. The conjugate of the present invention can be used for treating or diagnosis of a disease or a condition associated with expression of prostate specific membrane antigen (PSMA).

### Background

Hydroxypolymers are a group of sugar polymers, which constitute multiple hydroxyl groups, which can be activated and subsequently conveniently substituted with different moieties of effective compounds. Dextran is one of most frequently used hydroxypolymers in pharmaceutical industry with established pharmaceutical uses, e.g. for preventing hypovolemic chock, preventing embolism and improving microcirculation. Dextran and its non-toxic properties and high tolerability is very well documented (AS Segal, 1964 In: Modern medical monographs, ed. Wright, IS, Green & Stratton, NY, London, pp 5-17. Therefore, it is often used as an example of pharmaceutically applicable hydroxypolymers.

Guanidine compounds have many interesting and important biomedical effects and consequently many uses. They are for example used as free radical scavengers, inhibitors of nitric oxide synthase (NOS) preventing nitric oxide formation. They are known to have certain anti-proliferative effects on cells. The guanidines are also used as "anti-aging" drugs, wherein the effect is achieved by protection from low density lipoprotein oxidation, by prevention of atherosclerotic lesions formation, by retarding the tissue damaging effects of diabetes, e.g. cardiovascular damage, through inhibition of the formation of advanced glycosylation end products (AGE's). Aminoguanidine is currently supplied as a non-prescription drug in the United States and its role in the treatment of various diseases have been investigated in clinical studies.

Targeting or tumor cell selectivity is believed to be achieved through the increased metabolic needs of the fast dividing tumor cells, which require amino-5-guanidinopentanoic acid and structurally related guanidine compounds, and other constituents for polyamine formation (D Scott Lind, Am Soc Nutr Sci, 2004, J Nutr, 134:2837-2841; E Wayne, Turk J Med Sci, 2003, 33, 195-205). Cellular uptake of said guanidine compounds is mediated principally via the Na+ -independent basic amino acid transport system y+ (Cendan et al, Ann Surg Oncol, 1995, 2:257-265).

In European patent EP2131867 B1 the present inventors demonstrated that although guanidine compounds and dextrans, separately used have almost no toxic effect on tumor cells, guanidine dextran conjugates demonstrated an antitumor activity that was similar or at some dosages even higher than that of convention antitumor drugs, like Adriamycin®.

Another present inventor's European patent EP1137441 B1 discloses the use of a compound comprising dextran, the charge of which has been modified through covalent binding of charged lysine groups (lysine-dextran), for the manufacture of a medicament for treating charged tumors by selective targeting.

Still another European patent EP2274018 B1 of the present inventor is related to a modified hydroxypolymer conjugate, preferably a dextran-guanidine-biphosphonate conjugate for treating not only skeletal tumors i.e. bone metastasis, particularly bone metastasis related to hormone refractory prostate cancer HRPC and breast cancer, but also osteoporosis. Prostate-specific Membrane Antigen (PSMA), also known as glutamate carboxypeptidase II and N-acetylated alpha-linked acidic dipeptidase 1, is a dimeric type II transmembrane glycoprotein belonging to the M28 peptidase family encoded by the gene FOLH1 (folate hydrolase 1). The protein acts as a glutamate carboxypeptidase on different alternative substrates, including the nutrient folate and the neuropeptide N-acetyl-1-aspartyl-1-glutamate and is expressed in a number of tissues such as the prostate, and to a lesser extent, the small intestine, central and peripheral nervous system and kidney. PSMA is present on the neovasculature of a wide range of malignant tumors including: prostate, renal, pancreatic, breast, colon, bladder, testicular carcinoma, melanoma, glioblastoma, and soft tissue sarcomas. PSMA has been shown to be an important target for immunological approaches such as vaccines or directed therapy with monoclonal antibodies. Unlike other prostate-restricted molecules that are secretory proteins (PSA, prostatic acid phosphatase), PSMA is an integral cell-surface membrane protein that is not secreted, which makes it an ideal target for antibody therapy. Radioactive molecules that selectively bind to specific tumor cell surface proteins provide an attractive route for imaging and treating tumors under non-invasive conditions. In particular, radiolabeled ligands to the PSMA protein that is often overexpressed on many cancer cells, provide an attractive route for non-invasive imaging and selective targeting of cancer cells.

PROSTASCINT@ (capromab pendetide) is an In-labelled anti-PSMA murine monoclonal antibody approved by the FDA for imaging and staging of newly diagnosed and recurrent prostate cancer patients. Capromab binds to an intracellular epitope of PSMA, thus requiring internalization or exposure of the internal domain of PSMA, therefore preferentially binding apoptotic or necrosing cells. Also monoclonal antibodies that bind to the extracellular domain of PSMA and have been radiolabeled and shown to accumulate in PSMA-positive prostate tumor models in animals. However, diagnosis and tumor detection using monoclonal antibodies has been limited by the low permeability of the monoclonal antibody in solid tumor.

While monoclonal antibodies hold promise for tumor detection and therapy, there have been limited clinical successes outside of lymphoma because of their low permeability in solid tumors. Low molecular weight mimetics, with higher permeability in solid tumors will have a definite advantage in obtaining high percent per gram and a high percentage of specific binding.

EP 2097111 B1 discloses glutamate-urea-lysine PSMA-binding moiety conjugated to a radio-imaging moiety that enables rapid visualization of prostate cancer and specific targeting to allow radiotherapy. Further PSMA ligands/modulators are disclosed in WO2015/058151 A2, WO2014/127365 A1 and EP3011976 A1.

PSMA is thus recognized as an attractive molecular target for the development of radiopharmaceuticals to image and treat metastatic prostate cancer but also other cancers wherein PSMA is expressed.

### Brief Description of the Invention

The present invention is related to modified dextran conjugates that comprise a lysine-urea-glutamate pharmacophore.

The present invention is also related to a tumor killing composition which comprises the modified dextran conjugate having the lysine-urea-glutamate pharmacophore and at least one pharmaceutically acceptable adjuvant.

The present invention further comprises a pharmaceutical formulation, comprising the modified dextran conjugate encompassing the lysine-urea-glutamate pharmacophore, a pharmaceutically acceptable salt or solvate thereof, and a pharmaceutically acceptable excipient.

Also a method for treating cancer in a subject, wherein said method comprises administering to a subject in need of such treatment a therapeutically effective amount of the tumor killing composition comprising the modified dextran conjugate containing the lysine-urea-glutamate pharmacophore and at least one pharmaceutically acceptable adjuvant or the pharmaceutical formulation comprising the modified dextran conjugate having the lysine-urea-glutamate pharmacophore, a pharmaceutically acceptable salt or solvate thereof, and a pharmaceutically acceptable excipient, is disclosed.

Further, the present invention relates to a method for producing the modified dextran conjugate containing the lysine-urea-glutamate pharmacophore, wherein a guanidine compound having at least one free amino group is mixed with activated dextran and glutamine-urea-ly sine.

### Brief Description of the Figures

**Figure 1** shows the principal molecular structure obtained with the chemistry of the present invention. The structure Lig_PSMA-DX is glutamate-urea-lysine-dx conjugate, wherein dx symbols guanidine-dextran.

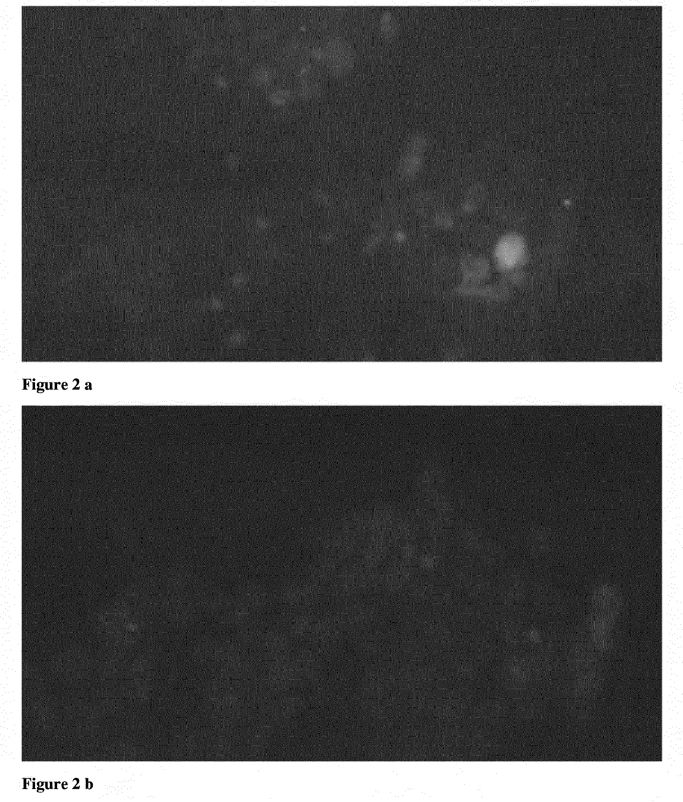

**Figure 2** a) shows Lig_PSMA-DX_FITC conjugate binding to PSMA positive 22Rv1 tumor cells and b) demonstrates that Lig_PSMA-DX-FITC conjugate does not bind to DU145 cells that do not express PSMA.
**Figure 3** a) demonstrates Lig_PSMA-DX_FITC conjugate binding in 22Rv1 cells expressing PSMA and b) demonstrates the inhibition of Lig_PSMA-DX-FITC conjugate binding when excess amount of Lig_PSMA (i.e. free glu-urea-lys) is present in 22Rv1 cells with PSMA expression.

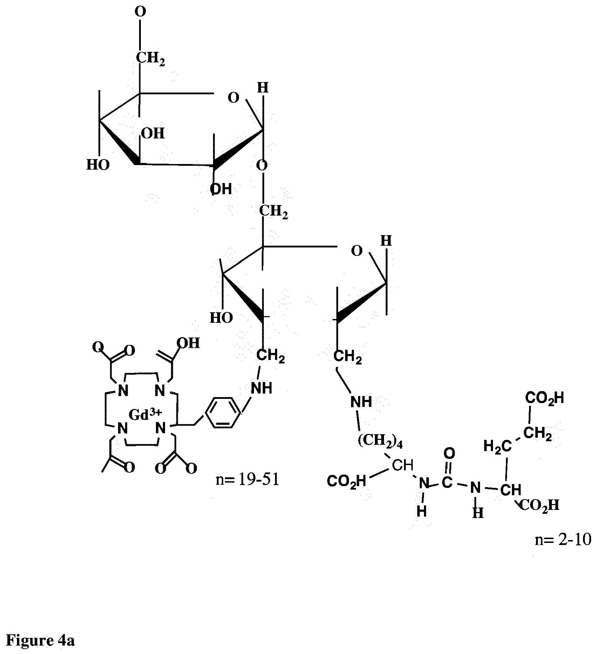

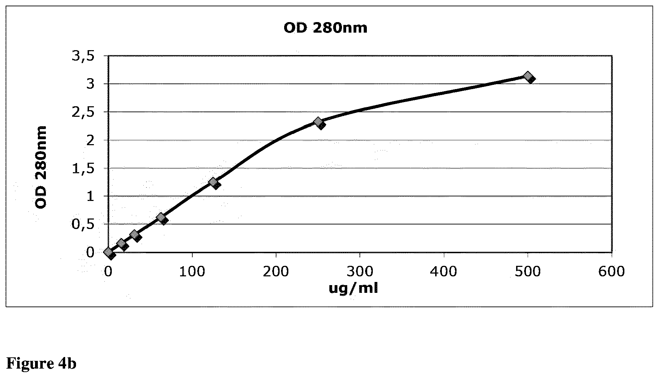

**Figure 4** a) shows the molecular structure of the gadolinium labelled 2-(4-aminobenzyl)-1,4,7 10-tetraazacyclododecanetetraacetic acid (Gd-DOTA) conjugated to dextran-Lys-Urea-Glut. As indicated, the substitution degree can be modulated for both ligands depending on the application/purpose and b) shows a spectrophotometric standard curve at A280 prepared for Gd-DOTA for the determination of degree of conjugation.

### Detailed Description of the Invention

It has been earlier shown that some compounds containing a glutamate-urea-glutamate (GUG) or a glutamate-urea-lysine (GUL) recognition element linked to a radionuclide-ligand conjugate exhibit high affinity for PSMA. The present inventors have invented a conjugate, wherein the PSMA protein binding glutamate-urea-lysine tripeptide is combined with modified dextran that can further be combined with compounds having free amino group. These compounds include various ligands, such as cytostatic agents or diagnostic/therapeutic radionuclides. The modified dextran conjugate of the present invention provides an attractive route for non-invasive imaging, selective targeting and therapy of PSMA expressing cancers. The present invention relates to a modified dextran conjugate encompassing a lysine-urea-glutamate pharmacophore. According to one preferred embodiment the conjugate is a PSMA binding conjugate. According to another preferred embodiment, the dextran moiety is substituted with a compound which have at least one free amino group. According to further preferred embodiment the dextran moiety is substituted with a guanidine compound. According to one preferred embodiment the conjugate further comprises substitution with other ligands in the dextran moiety. In a further aspect of the invention the other ligand is selected from the group consisting of therapeutic compounds, cytostatic drugs and radiometal chelators that may chelate gamma, beta or alpha emitting radionuclides. In one preferred embodiment the radionuclide is a gamma emitting (technetium-99m)Tc(CO)3 chelate complex, beta emitting (rhenium-186/188)Re(CO)3 chelate complex or alpha emitting radionuclides chelate complex.

According to another embodiment the dextran moiety is substituted with a gadolinium labelled metal chelate. The substitution with gadolinium metal chelate does not reduce the function of the conjugate as compared e.g. to the situation, wherein the dextran moiety is substituted with a guanidine compound.

One compound that can be added to the modified dextran conjugate encompassing a lysine-urea-glutamate pharmacophore is 1,4,7,10-tetraazacyclododecane-1,4,7,10-tetraacetic acid (also known as DOTA), which is an organic compound with the formula (CH₂CH₂NCH₂CO₂H)₄. The molecule consists of a central 12-membered tetraaza (i.e., containing four nitrogen atoms) ring. DOTA is used as a complexing agent, especially for lanthanide ions. Its complexes have medical applications as contrast agents and cancer treatments.

The acronym DOTA is shorthand for both the tetracarboxylic acid and its various conjugate bases. In the area of coordination chemistry, the tetraacid is called H₄DOTA and its fully deprotonated derivative is DOTA⁴⁻. Many related ligands are referred to using the DOTA acronym, although these derivatives are generally not tetracarboxylic acids or the conjugate bases.

DOTA is derived from the macrocycle known as cyclen. The four secondary amine groups are modified by replacement of the N-H centers with N-CH2CO2H groups. The resulting aminopolycarboxylic acid, upon ionization of the carboxylic acid groups, is a high affinity chelating agent for di- and trivalent cations. As a polydentate ligand, DOTA envelops metal cations, but the denticity of the ligand depends on the geometric tendencies of the metal cation. The main applications involve the lanthanides and in such complexes DOTA functions as an octadentate ligand, binding the metal through four amine and four carboxylate groups. Most such complexes feature an additional water ligand, giving an overall coordination number of nine.

For most transition metals, DOTA functions as a hexadentate ligand, binding through the four nitrogen and two carboxylate centres. The complexes have octahedral coordination geometry, with two pendent carboxylate groups. In the case of [Fe(DOTA)]⁻, the ligand is heptadentate. DOTA was once used as part of some cancer therapies, where it functioned as a chelating agent for the radioisotope ⁹⁰Y³⁺. The research was discontinued when scientists found that the cancer only spread. DOTA can be conjugated to monoclonal antibodies by attachment of one of the four carboxyl groups as an amide. The remaining three carboxylate anions are available for binding to the yttrium ion. The modified antibody accumulates in the tumour cells, concentrating the effects of the radioactivity of ⁹⁰Y. Drugs containing this module receive an International Nonproprietary Name ending in tetraxetan (Yttrium (⁹⁰Y) clivatuzumab tetraxetan and Yttrium (⁹⁰Y) tacatuzumab tetraxetan).

DOTA can also be linked to molecules that have affinity for various structures. The resulting compounds are used with a number of radioisotopes in cancer therapy and diagnosis (for example in positron emission tomography).

According to one preferred embodiment the conjugate is administrated systemically i.e. by intravenous administration. The conjugate can be administered locally or regiolocally e.g. by administration to the peritoneal cavity. Also intravenous administration can be used for systemic administration. According to another preferred embodiment the conjugate is for use in the treatment, imaging or diagnosis of cancer. According to still another preferred embodiment said cancer comprises malignant tumors including prostate, renal, pancreatic, breast, colon, bladder, testicular carcinoma, melanoma, glioblastoma, and soft tissue sarcomas. In the most preferred embodiment said cancer is a prostate cancer.

The invention relates also to a tumor killing composition which comprises the modified dextran conjugate encompassing a lysine-urea-glutamate pharmacophore and at least one pharmaceutically acceptable adjuvant.

The invention further relates to a pharmaceutical formulation which comprises the modified dextran conjugate encompassing a lysine-urea-glutamate pharmacophore, a pharmaceutically acceptable salt or solvate thereof, and a pharmaceutically acceptable excipient.

Also a method for treating cancer in a subject, said method comprising administering to a subject in need of such treatment a therapeutically effective amount of the composition which comprises the modified dextran conjugate encompassing a lysine-urea-glutamate pharmacophore and at least one pharmaceutically acceptable adjuvant or the formulation which comprises the modified dextran conjugate encompassing a lysine-urea-glutamate pharmacophore, a pharmaceutically acceptable salt or solvate thereof, and a pharmaceutically acceptable excipient, is one aspect of the invention.

Another aspect of the invention is a method for producing the modified dextran conjugate as described above encompassing a lysine-urea-glutamate pharmacophore, wherein a guanidine compound having at least one free amino group is mixed with activated dextran and glutamate-urea-ly sine.

Earlier attempts to improve the effectivity of the PSMA binding tripeptide containing substances have focused on modifying the tripeptide. Differently to earlier known monovalent conjugates utilizing PSMA binding tripeptide the present invention with modified dextran component is a polyvalent construct providing superior avidity and thus offering several possibilities for targeting for example cytostatic agents or diagnostic and/or therapeutic radionuclides to PSMA expressing tumor cells.

Thus, in general, the focus of research on the construction of ligands intended for targeting of PSMA expressing malignant diseases has so far been entirely concentrated on monomeric modification of the PSMA binding pharmacophore Glutamate-Urea-Lysine. The most common modifications thereof are substitutions with one single radiometal chelator through chemical linking via the free amine of the pharmacophore i.e. the lysine epsilon amino group. The possibility of polymeric modification of the pharmacophore and its inherent advantages has simply been overlooked which is confirmed when reviewing related scientific publications and patent applications.

As used herein, the term "**polyvalent**" means that more than one type or class of ligand molecule can simultaneously be combined with the modified dextran conjugate. In the present invention, the ligands can be combined to the dextran moiety and/or, as traditionally done in constructs with the PSMA binding tripeptide, to the tripeptide.

In accordance with the invention, the phrases "**specifically targeting**", "**specific targeting**", and "**specifically targeted**" mean that the PSMA binding ligand conjugates described herein are preferentially targeted to prostate cancer cells or to other cells that preferentially express or overexpress PSMA as evidenced by the ability to detect accumulation of the PSMA binding ligand conjugates in the specifically targeted cell type over accumulation in normal tissues that do not express the receptor for the ligand.

The principle of targeting i.e. intravenous administration of a targeting molecule having a ligand, or ligands, that has a specificity for a specific structure or protein that is expressed by the target tumor cells, depends on the random collision between the targeting molecule and the target structure enabling the specific binding of the ligand. The significance of a polyvalent targeting molecule that has multiple specific ligands, is that the probability of binding to its target is increased. This increased binding signifies that the targeting is enhanced i.e. the diagnostic or therapeutic efforts become more effective.

The dextran moiety can be substituted with a compound which has at least one free amino group and which are covalently coupled to activated hydroxyl groups of the dextran moiety. The activated dextran polymer can be substituted for example by guanidine side groups of guanidine. The guanidine groups are covalently coupled to said dextran polymer via free amino side groups of the guanidine compounds. The substitution is preceded by activating said dextran polymer through oxidation, which enables the reaction with said free amino side groups forming bonds of guanidine compounds with subsequent reduction obtain stable amine bonds (Matsunaga et al, Nucl Med Biol. 2005, 32, 279-285). Term "**activated dextran**" as used herein thus refers to a product that results from oxidation of dextran in the presence of sodium periodate.

Alternatively to guanidine, the activated dextran polymer can also be coupled for example to several amino acids, such as lysine or tyrosine. The critical feature of these alternative elements to be coupled to the activated dextran polymer is the presence of a free amino group. In general, "**substituted**" refers to an alkyl or alkenyl group, as defined below (e.g., an alkyl group) in which one or more bonds to a hydrogen atom contained therein are replaced by a bond to non-hydrogen or non-carbon atoms. Substituted groups also include groups in which one or more bonds to a carbon(s) or hydrogen(s) atom are replaced by one or more bonds, including double or triple bonds, to a heteroatom. Thus, a substituted group will be substituted with one or more substituents, unless otherwise specified. In some embodiments, a substituted group is substituted with 1, 2, 3, 4, 5, or 6 substituents. Examples of substituent groups include: halogens (i.e., F, Cl, Br, and I); hydroxyls; alkoxy, alkenoxy, alkynoxy, aryloxy, aralkyloxy, heterocyclyloxy, and heterocyclylalkoxy groups; carbonyls (oxo); carboxyls; esters; urethanes; oximes; hydroxylamines; alkoxyamines; aralkoxyamines; thiols; sulfides; sulfoxides; sulfones; sulfonyls; sulfonamides; amines; N-oxides; hydrazines; hydrazides; hydrazones; azides; amides; ureas; amidines; guanidines; enamines; imides; isocyanates; isothiocyanates; cyanates; thiocyanates; imines; nitro groups; nitriles (i.e., CN), haloalkyl, aminoalkyl, hydroxyalkyl, cycloalkyl and the like. In a preferred embodiment the modified dextran is substituted with guanidine.

The guanidine-polymer-conjugate of the present invention is a compound which as a backbone compound has a hydroxylpolymer such as dextran. The molecular weight of hydroxylpolymer or dextran is between 10³-10⁶ Daltons. Typically, the hydroxypolymer or dextran is substituted with between 15 to 60%, preferably between 20 to 50% guanidine side groups, i.e. 20-50% of the glucose moieties in the dextran backbone are substituted with guanidine groups. In the exemplified case, the aminoguanidine forms a hydrazone bond with the hydroxypolymer or dextran polymer. The hydrazone bond can be further reduced, yielding a guanidino hydrazine bond. Suitable guanidine compounds are agmatine and aminoguanidine. The scope of the present invention also encompasses a ligand-linker complex that binds to PSMA and the use of this ligand-linker complex to target drugs to PSMA positive cells wherein the ligand is not a monoclonal antibody. The present invention also includes an anticancer moiety or radionuclide chelator that is covalently linked to the dextran backbone via a free amine e.g. 2-amino-benzyl.

The term "**chelating agent**", or "**chelator**", refers to a molecule, often an organic one, and often a Lewis base, having two or more unshared electron pairs available for donation to a metal ion. The metal ion is usually coordinated by two or more electron pairs to the chelating agent. The terms, "bidentate chelating agent", "tridentate chelating agent", and "tetradentate chelating agent" are art-recognized and refer to chelating agents having, respectively, two, three, and four electron pairs readily available for simultaneous donation to a metal ion coordinated by the chelating agent. Usually, the electron pairs of a chelating agent forms coordinate bonds with a single metal ion; however, in certain examples, a chelating agent may form coordinate bonds with more than one metal ion, with a variety of binding modes being possible.

Stable attachment of radionuclides by means of macrocylic chelating agents to targeting agents results in agents for targeted radiotherapy/diagnosis. The chelators that hold the radionuclide have to have high stability at physiologic conditions to avoid radiation damage to non-target cells. When constructing such agents it is essential to perform thorough optimization of degree of substitution and the stability of the construct. The addition of a 2-amino-benzyl group to the macrocylic radiometal chelator results in a chemical handle, the NH2 group, and a chromophore, the benzyl. The chemical handle is excellent for reductive amination and the chromphore greately facilitates the optimization of the construction. This is done by the making of a spectrophotometric standard curve at A280 and then determination e.g. substitution degree, i.e. degree of coupling of the macrocylcic chelator, can be performed at A280 directly after synthesis of each derivative of targeting agent.

According to the present invention modified dextran conjugates comprising glutamate-urea-lysine tripeptide may also have substitution of other ligands, e.g., cytostatic compounds, radiometal chelators that may chelate gamma, beta or alpha emitting radionuclides for cancer diagnosis or therapy. Also lysine or any other amino acid or a derivative thereof may be conjugated depending on the purpose.

Other functional groups may be coupled additionally to guanidine-dextran-lysine-urea-glutamate conjugate. Those compounds include chemotherapeutic drugs, radionuclides, for example, Tc-99m, 1-131, Y-90, Re-188, Sm-153, hormones, hormone antagonists, such as Tamoxifen, peptides, such as somatostatin, toxins, monoclonal antibodies or fragments thereof, free radical scavengers, such as amifostine or proteins. Coupling of such functional groups could be achieved directly or via bifunctional chelates that have been coupled to the hydroxypolymer prior to the inclusion of functional groups.

Radionuclides or radiometals comprise many useful radioactive isotopes of various metallic elements. When properly harnessed, these have valuable emission properties that can be used for diagnostic imaging techniques, such as single photon emission computed tomography (SPECT, e.g.(67)Ga, (99m)Tc, (111)In, (177)Lu) and positron emission tomography (PET, e.g.(68)Ga, (64)Cu, (44)Sc, (86)Y, (89)Zr), as well as therapeutic applications (e.g.(47)Sc, (114m)In, (177)Lu, (90)Y, (212/213)Bi, (212)Pb, (225)Ac, (186/188)Re). A fundamental critical component of a radiometal-based radiopharmaceutical is the chelator, the ligand system that binds the radiometal ion in a tight stable coordination complex so that it can be properly directed to a desirable molecular target in vivo. In the present invention, chelators should have a free amine group for the conjugation, preferably a 2-amino-benzyl group allowing spectrophotometric determination of the content of chelate in the construct. Illustrative chemotherapeutic drugs and agents include, but are not limited to, adrenocorticoids and corticosteroids, alkylating agents, antiandrogens, antiestrogens, androgens, aclamycin and aclamycin derivatives, estrogens, antimetabolites such as cytosine arabinoside, purine analogs, pyrimidine analogs, and methotrexate, busulfan, carboplatin, chlorambucil, cisplatin and other platinum compounds, tamoxiphen, taxol, paclitaxel, paclitaxel derivatives, Taxotere®, cyclophosphamide, daunomycin, rhizoxin, T2 toxin, plant alkaloids, prednisone, hydroxyurea, teniposide, mitomycins, discodermolides, microtubule inhibitors, epothilones, tubulysins, cyclopropyl benz[e]indolone, secocyclopropyl benz[e]indolone, O-Ac-secocyclopropyl benz[e]indolone, bleomycin and any other antibiotic, nitrogen mustards, nitrosureas, vinca alkaloids, such as vincristine, vinblastine, vindesine, vinorelbine and analogs and derivative thereof such as deacetylvinblastine monohydrazide (DAVLBH), colchicine, colchicine derivatives, allocolchicine, thiocolchicine, trityl cysteine, halicondrin B, dolastatins such as dolastatin 10, amanitins such as a-amanitin, camptothecin, irinotecan, and other camptothecin derivatives thereof, geldanamycin and geldanamycin derivatives, estramustine, nocodazole, MAP4, colcemid, inflammatory and proinflammatory agents, peptide and peptidomimetic signal transduction inhibitors, rapamycins, such as sirolimus and everolimus, and any other drug or toxin. In order to the chemotherapeutic drugs and agents to be conjugated to the conjugate according to the invention, there has to be a free amine group, naturally or introduced by chemical modification, present in said drug or agent.

Term "**pharmacophore**" as used herein is an abstract description of molecular features which are necessary for molecular recognition of a ligand by a biological macromolecule. The IUPAC defines a pharmacophore to be "an ensemble of steric and electronic features that is necessary to ensure the optimal supramolecular interactions with a specific biological target and to trigger (or block) its biological response". Typical pharmacophore features include hydrophobic centroids, aromatic rings, hydrogen bond acceptors or donors, cations, and anions. The features need to match different chemical groups with similar properties, in order to identify novel ligands. The pharmacophore used in the present invention is the glutamate-urea-lysine tripeptide.

An aspect of the present invention is to use guanidine-dextran-glutamate-urea-lysine PSMA binding conjugate for treatment and diagnosis of malignant tumors including: prostate, renal, pancreatic, breast, colon, bladder, testicular carcinoma, melanoma, glioblastoma, and soft tissue sarcomas. Preferably, the malignant tumor is a prostate tumor.

The present invention can be used for diagnosis and/or treatment of PSMA expressing tumors, especially prostate cancer.

A tumor is a commonly used, but non-specific, term for a neoplasm. The word tumor simply refers to a mass. This is a general term that can refer to benign (generally harmless) or malignant (cancerous) growths. A cancer is another word for a malignant tumor (a malignant neoplasm). Terms "**cancer**" and "**tumor**" are used herein as synonyms and interchangeably. Another aspect of the invention is a method for producing the guanidine-dextran-glutamate-urea-lysine PSMA binding conjugate wherein a guanidine compound having at least one free amino group is mixed with activated dextran and glutamine-urea-lysine.

Still a further aspect of the invention is a pharmaceutical formulation, comprising the guanidine-dextran-glutamate-urea-lysine PSMA binding conjugate, a pharmaceutically acceptable salt or solvate thereof, and a pharmaceutically acceptable excipient.

One aspect of the invention is also a method of treatment, wherein modified dextran conjugate comprising the glutamate-urea-lysine PSMA binding pharmacophore is administered to a patient in need thereof in an effective amount.

The phrases "**effective amount**" or "**therapeutically-effective amount**" as used herein means that amount of a compound, material, or composition comprising a compound or conjugate of the invention, or other active ingredient which is effective for producing some desired therapeutic effect in at least a sub-population of cells in an animal at a reasonable benefit/risk ratio applicable to any medical treatment. A therapeutically effective amount with respect to a compound of the invention means that amount of therapeutic agent alone, or in combination with other therapies, that provides a therapeutic benefit in the treatment or prevention of a disease. Used in connection with a compound of the invention, the term can encompass an amount that improves overall therapy, reduces or avoids symptoms or causes of disease, or enhances the therapeutic efficacy of or synergies with another therapeutic agent.

As used herein, the terms "**treating**" or "**treatment**" is intended to encompass also diagnosis, prophylaxis, therapy and cure. The patient receiving this treatment is any animal in need, including primates, in particular humans, and other mammals such as equines, cattle, swine and sheep; and poultry and pets in general.

A further aspect of the present invention is a tumor killing composition, which comprises the modified dextran conjugate comprising the glutamate-urea-lysine PSMA binding pharmacophore and at least one pharmaceutically acceptable adjuvant. Examples of suitable adjuvants are a therapeutic radionuclide and an anthracycline.

In another aspect, the compositions comprising conjugates according to the invention include a therapeutically effective amount of the one or more conjugates or compounds for diagnosis, imaging, and/or treatment of diseases caused by PSMA expressing cells in a patient. Illustrative compositions include unit doses, unit dosage forms, and the like. It is to be understood that the compositions comprising conjugate according to the invention may include other components and/or ingredients, including, but not limited to, other therapeutically active compounds, and/or one or more carriers, and/or one or more diluents, and/or one or more excipients, and the like. In another embodiment, methods for using the compounds and pharmaceutical compositions for diagnosis, imaging, and/or treatment of diseases caused by PSMA expressing cells in a patient are also described herein.

In one aspect, the methods include the step of administering one or more conjugates described herein to the patient. In another embodiment, uses of the compounds and compositions in the manufacture of a medicament for diagnosis, imaging, and/or treatment of diseases caused by PSMA expressing cells in a patient are also described herein. In one aspect, the medicaments include a therapeutically effective amount of the one or more compounds and/or compositions described herein.

The modified dextran conjugate of the invention has a three-step mechanism of action comprising binding, internalization and exerting the therapeutic effect. The guanidine moieties facilitate targeting to the cancer cells through their electrostatic charge, the pharmacophore binds to PSMA, and the therapeutic moiety produces its effects after internalization into the cancer cell.

It is appreciated herein that the conjugates and compositions described herein may be used alone or in combination with other compounds useful for diagnosis, imaging, and/or treatment of diseases caused by PSMA expressing cells in a patient, including those compounds that may be therapeutically effective by the same or different modes of action. In addition, it is appreciated herein that the conjugates and compositions described herein may be used in combination with other compounds that are administered to treat other symptoms of the disease, such as compounds administered to decrease pain, and the like.

The medicine is preferably administered systemically, i.e. by intra-venous administration. The medicine can be administered locally or regiolocally, e.g. by administration to the peritoneal cavity. All these routes of administration are possible and dependent on the specific therapeutic application.

There currently exists a critical unmet medical need for new imaging modalities to assist physicians in selecting appropriate treatment regimens for prostate cancer and improving patient outcomes. The inventors believe that the PSMA binding conjugates according to the present invention, which may be capable of detecting both the primary prostate cancer as well as soft tissue and bone metastases, will not only satisfy this critical unmet need, but could alter the current paradigm for the detection and staging of prostate cancer and also offer a unique opportunity to follow response to systemic therapies by non-invasive external imaging. Patient management would be significantly improved as these molecular imaging pharmaceuticals are designed to track specifically both the location and progression of prostate tumor metastases through their PSMA expression.

The especial advantage of the present invention is that the same platform can be used for both diagnosis and treatment. The use of the modified dextran conjugate of the present invention allows an earlier and more accurate diagnosis and represents therapeutic options which, truly allow to affect the biology of the disease in an advanced and metastatic stage so as to more effectively cope with an ever increasing social and medical problem that has uncertain solution perspectives with current therapeutical measures.

### Example 1

### Conjugate synthesis

### Materials

Dextran 40 (PhEUR; 10kD- 500kD, preferable 40kD or 70kD) (Pharmacosmos AS, Denmark) was used as conjugate backbone. Sodium metaperiodate (Merck AG, Darmstadt, Germany) was used for oxidation/activation). Aminoguanidine, L-Lysine (Sigma-Aldrich, Sweden) and the PSMA ligand Glutamate-Urea-Lysine (Peptides & Elephants, custom synthesis) were used for the conjugation. Sodium cyanoborohydride (Sigma Aldrich, Stockholm, Sweden) was used for reductive amination. PD-10 disposable Sephadex G-25 columns were used for separation and purification (G&E Biotech AB, Sweden). FITC was from Sigma Aldrich.

### Activation and conjugation

30mg of dextran was mixed with 18mg of sodiumperiodate in 1 ml of destilled H₂O and the pH was adjusted to 1.6 with H₂SO₄. The mixture was incubated on a magnetic stirrer for 45 min at RT in the dark.

### Conjugation

30 mg of activated dextran was mixed with 20 mg Glut-Urea-Lysine in 1 ml 0.2 M Sodiumphosphate buffer at pH 7.4 + 5 mg sodiumcyanoborohydride and then incubated in the dark at RT for 120 min on a magnetic stirrer. After 120 min, 12 mg aminoguanidine and 12 mg Lysine was added and the incubation continued for an additional 60 min. After 4 h of total incubation, the solution was then purified on a PD-10 column using 20 mM Borate buffer at pH 9.5 as eluent.

### FITC labelling

15 mg of conjugate in 1mL borate buffer at pH 9.5 was mixed with 50 µg FITC and incubated in the dark at RT for 18 h. After incubation, the conjugate was purified on a PD10 column and eluted in sodiumacetate at pH 6.

### Cell Line and Culture Conditions

The human metastatic prostate carcinoma cell lines 22Rv1, DU145 and PC3 obtained from American Type Culture Collection (ATCC, USA) was used. The cell line was cultured in 5 ml dishes (Falcon) with RPMI 1640 (Sigma). 2 mM glutamine (Sigma), 10 % fetal calf serum (Life Technologist) and 1 % penicillin/streptomycin (Gibco) were added to the media. The cells were kept in a 37 °C incubator with humidified air and 5 % CO2. The culture medium was changed two times a week. Cells were removed from the dishes by treatment trypsin(0.5g)-EDTA(0.2g)(Sigma, USA).

The cells were seeded on 8 chamber glasses 50 000 in each well, 2 wells for each cell line. After 24 h in media in the incubator the cells were rinsed in phosphate buffered saline (PBS) and incubated with Lig_PSMA-Dx-FITC conjugate with or without blocking/inhibition with PSMA-ligand. All incubations were performed in PBS at RT. The PSMA ligand at a concentration of 627 µM was used to inhibit the Lig_PSMA-Dx-FITC conjugate PSMA binding. After 10 min the Lig_PSMA-Dx-FITC conjugate was added at a concentration of 1.25 µM. The cells without blocking were only treated with Lig_PSMA-Dx-FITC 1.25 µM for 10 min.

After another 10 min the cells were rinsed carefully in PBS and fixed in 4 % formaldehyde for 10 min. The glasses were then mounted with mounting media with DAPI (Vectashield). The glasses were analysed in a Zeiss microscope and photos were obtained using Axiovision software.

### Results

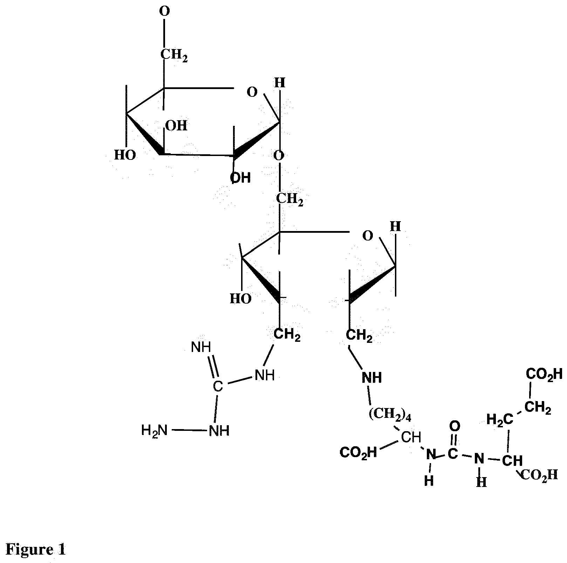

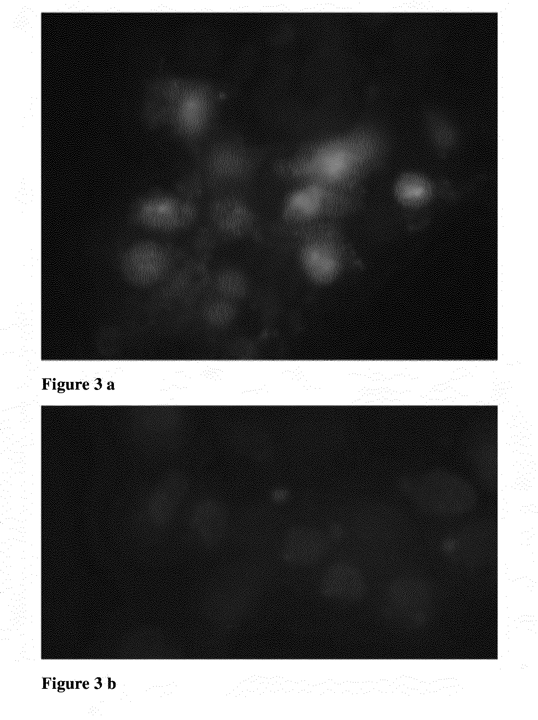

The examples serve to demonstrate that guanidine-dextran-glutamate-urea-lysine PSMA binding conjugate according to the invention is selectively accumulated in tumor cells. Figure 1a shows that Lig_PSMA-DX_FITC conjugate is able to bind to PSMA positive tumor cells 22Rv1, while Figure 1b demonstrates that Lig_PSMA-DX-FITC conjugate does not bind to DU145 cells that do not express PSMA. Figure 3 further shows that the binding of Lig_PSMA-DX_FITC conjugate to PSMA positive tumor cells 22Rv1 can be inhibited by excess amount of free glutamine-urea-lysine.

### Example 2

### Activation and conjugation

30mg of dextran was mixed with 18mg of sodiumperiodate in 1 ml of destilled H₂O and the pH was adjusted to 1.6 with H2SO4. The mixture was incubated on a magnetic stirrer for 45 min at RT in the dark. After activation, the mixture was purified on PD10 sephadex column and the purified and activated dextran was eluted in 0.2M sodiumacetate buffer at pH 6,5.

### Conjugation

30 mg of activated dextran was mixed with 40 mg Glu-Urea-Lysine in 2 ml 0.2 M Sodiumacetate buffer at pH 6.4 + 10 mg sodium cyanoborohydride and then incubated in the dark at RT for 180 min on a magnetic stirrer. After 180 min, 40mg DOTA was added and the incubation continued overnight. After incubation, the solution was then purified on two PD-10 columns using 0.2M sodium acetate buffer at pH 6 as eluent (1mL mixture purified on each column). A sample from the purified conjugate was taken and measured at a spectrophotometer at A280. The absorbancy was noted and compared to the DOTA standard curve and the substitution degree could be calculated.

## Claims

1. A modified dextran conjugate, **characterized in that** the conjugate comprises lysine-urea-glutamate pharmacophore.

2. The modified dextran conjugate according to claim 1, **characterized by** that the dextran moiety is substituted with a compound which have at least one free amino group.

3. The modified dextran conjugate according to claim 2, **characterized by** that the dextran moiety is substituted with a guanidine compound.

4. The modified dextran conjugate according to claim 2, **characterized by** that the dextran moiety is substituted with a gadolinium labelled metal chelate.

5. The modified dextran conjugate according to any of claims 1 to 4, **characterized by** that the conjugate further comprises substitution with other ligands in the dextran moiety.

6. The modified dextran conjugate according to claim 5, **characterized in that** the other ligand is selected from the group consisting of therapeutic compounds, cytostatic drugs and radiometal chelators that may chelate gamma, beta or alpha emitting radionuclides.

7. The modified dextran conjugate according to claim 6, **characterized in that** the radionuclide is a gamma emitting (technetium-99m)Tc(CO)3 chelate complex, beta emitting (rhenium-186/188)Re(CO)3 chelate complex or alpha emitting radionuclides chelate complex.

8. The modified dextran conjugate according to any of claims 1 to 7, **characterized in that** said conjugate is a prostate specific membrane antigen (PSMA) binding conjugate.

9. The modified dextran conjugate according to any of claims 1 to 8 for use in the treatment, imaging or diagnosis of cancer.

10. The modified dextran conjugate for use according to claim 9, **characterized in that** said cancer comprises malignant tumors including prostate, renal, pancreatic, breast, colon, bladder, testicular carcinoma, melanoma, glioblastoma, and soft tissue sarcomas.

11. The modified dextran conjugate for use according to claim 10, **characterized in that** said cancer is a prostate cancer.

12. A tumor killing composition, **characterized in that** said tumor killing composition comprises the modified dextran conjugate according to any of claims 1 to 8 and at least one pharmaceutically acceptable adjuvant.

13. A pharmaceutical formulation, comprising the modified dextran conjugate according to any of claims 1 to 8, a pharmaceutically acceptable salt or solvate thereof, and a pharmaceutically acceptable excipient.

14. A compound for use in a method for treating cancer in a subject, said method comprising administering to a subject in need of such treatment a therapeutically effective amount of the composition according to claim 12 or the formulation according to claim 13.

15. A method for producing the modified dextran conjugate according to any of claims 1 to 3 or 5 to 8, **characterized in that** a guanidine compound having at least one free amino group is mixed with activated dextran and glutamine-urea-lysine.

## Patentansprüche

1. Modifiziertes Dextrankonjugat, **gekennzeichnet dadurch, dass** das Konjugat ein Lysin-Harnstoff-Glutamat-Pharmakophor umfasst.

2. Modifiziertes Dextrankonjugat nach Anspruch 1, **gekennzeichnet dadurch, dass** der Dextran-Anteil mit einer Verbindung, die mindestens eine freie Aminogruppe aufweist, substituiert wird.

3. Modifiziertes Dextrankonjugat nach Anspruch 2, **gekennzeichnet dadurch, dass** der Dextran-Anteil mit einer Guanidinverbindung substituiert wird.

4. Modifiziertes Dextrankonjugat nach Anspruch 2, **gekennzeichnet dadurch, dass** der Dextran-Anteil mit einem Gadolinium-markierten Metallchelat substituiert wird.

5. Modifiziertes Dextrankonjugat nach einem der Ansprüche 1 bis 4, **gekennzeichnet dadurch, dass** das Konjugat ferner eine Substitution mit anderen Liganden im Dextran-Anteil umfasst.

6. Modifiziertes Dextrankonjugat nach Anspruch 5, **gekennzeichnet dadurch, dass** der andere Ligand ausgewählt ist aus der Gruppe bestehend aus therapeutischen Verbindungen, zytostatischen Arzneimitteln und Radiometall-Chelatbildnern, die Gamma-, Beta- oder Alpha-strahlende Radionuklide chelatieren können.

7. Modifiziertes Dextrankonjugat nach Anspruch 6, **gekennzeichnet dadurch, dass** das Radionuklid ein Gamma-strahlender (Technetium-99m)Tc(CO)3-Chelat-Komplex, ein Beta-strahlender (Rhenium-186/188)Re(CO)3-Chelat-Komplex oder ein Alphastrahlender Radionuklid-Chelat-Komplex ist.

8. Modifiziertes Dextrankonjugat nach einem der Ansprüche 1 bis 7, **gekennzeichnet dadurch, dass** das Konjugat ein das Prostata-spezifische Membran-Antigen (PSMA) bindendes Konjugat ist.

9. Modifiziertes Dextrankonjugat nach einem der Ansprüche 1 bis 8 zur Verwendung in der Behandlung, Bildaufnahme oder Diagnose von Krebs.

10. Modifiziertes Dextrankonjugat zur Verwendung nach Anspruch 9, **gekennzeichnet dadurch, dass** der Krebs bösartige Tumore umfasst, die Prostata-, Nieren-, Bauchspeicheldrüsen-, Brust-, Dickdarm-, Blasen-, Hodenkarzinome, -Melanome, -Glioblastome und Weichgewebesarkome umfassen.

11. Modifiziertes Dextrankonjugat zur Verwendung nach Anspruch 10, **gekennzeichnet dadurch, dass** der Krebs Prostatakrebs ist.

12. Tumorabtötende Zusammensetzung, **gekennzeichnet dadurch, dass** die tumorabtötende Zusammensetzung das modifizierte Dextrankonjugat nach einem der Ansprüche 1 bis 8 und mindestens einen pharmazeutisch verträglichen Hilfsstoff umfasst.

13. Pharmazeutische Formulierung, die das modifizierte Dextrankonjugat nach einem der Ansprüche 1 bis 8, ein pharmazeutisch verträgliches Salz oder Solvat eines derartigen Salzes und einen pharmazeutisch verträglichen Exzipienten umfasst.

14. Verbindung zur Verwendung in einem Verfahren zur Behandlung von Krebs bei einem Patienten, wobei das Verfahren umfasst: Verabreichen einer therapeutisch wirksamen Menge der Zusammensetzung nach Anspruch 12 oder der Formulierung nach Anspruch 13 an einen Patienten mit entsprechendem Behandlungsbedarf.

15. Verfahren zur Herstellung des modifizierten Dextrankonjugats nach einem der Ansprüche 1 bis 3 oder 5 bis 8, **gekennzeichnet dadurch, dass** eine Guanidinverbindung, die mindestens eine freie Aminogruppe aufweist, mit aktiviertem Dextran und Glutamin-Harnstoff-Lysin gemischt wird.

## Revendications

1. Conjugué de dextrane modifié, **caractérisé en ce que** le conjugué comprend un pharmacophore lysine-urée-glutamate.

2. Conjugué de dextrane modifié selon la revendication 1, **caractérisé en ce que** la partie dextrane est substituée avec un composé ayant au moins un groupe amino libre.

3. Conjugué de dextrane modifié selon la revendication 2, **caractérisé en ce que** la partie dextrane est substituée avec un composé de guanidine.

4. Conjugué de dextrane modifié selon la revendication 2, **caractérisé en ce que** la partie dextrane est substituée avec un chélate de métal marqué au gadolinium.

5. Conjugué de dextrane modifié selon l'une des revendications 1 à 4, **caractérisé en ce que** le conjugué comprend également une substitution avec d'autres ligands dans la partie dextrane.

6. Conjugué de dextrane modifié selon la revendication 5, **caractérisé en ce que** l'autre ligand est choisi dans le groupe constitué par des composés thérapeutiques, médicaments cytostatiques et chélateurs de radiométal pouvant chélater des radionucléides à émission gamma, bêta ou alpha.

7. Conjugué de dextrane modifié selon la revendication 6, **caractérisé en ce que** le radionucléide est un complexe chélaté de (technetium-99m)Tc(CO)3 à émission gamma, un complexe chélaté de (rhénium-186/188)Re(CO)3 à émission bêta ou un complexe chélaté de radionucléides à émission alpha.

8. Conjugué de dextrane modifié selon l'une des revendications 1 à 7, **caractérisé en ce que** ledit conjugué est un conjugué se liant à l'antigène membranaire spécifique de la prostate (PSMA).

9. Conjugué de dextrane modifié selon l'une des revendications 1 à 8 pour l'utilisation dans le traitement, l'imagerie ou le diagnostic d'un cancer.

10. Conjugué de dextrane modifié pour l'utilisation selon la revendication 9, **caractérisé en ce que** le cancer comprend des tumeurs malignes comprenant des carcinomes, mélanomes, glioblastomes de la prostate, du rein, du pancréas, du sein, du colon, de la vessie, des testicules, et des sarcomes de tissu mou.

11. Conjugué de dextrane modifié pour l'utilisation selon la revendication 10, **caractérisé en ce que** ledit cancer est un cancer de la prostate.

12. Composition tumoricide, **caractérisée en ce que** ladite composition tumoricide comprend le conjugué de dextrane modifié selon l'une des revendications 1 à 8 et au moins un adjuvant pharmaceutiquement acceptable.

13. Formulation pharmaceutique comprenant le conjugué de dextrane modifié selon l'une des revendications 1 à 8, un sel pharmaceutiquement acceptable ou solvate dudit sel et un excipient pharmaceutiquement acceptable.

14. Composé pour l'utilisation dans un procédé de traitement de cancer chez un patient, ledit procédé comprenant l'étape consistant à administrer à un patient nécessitant un tel traitement une quantité thérapeutiquement efficace de la composition selon la revendication 12 ou de la formulation selon la revendication 13.

15. Procédé de fabrication du conjugué de dextrane modifié selon l'une des revendications 1 à 3 ou 5 à 8, **caractérisé en ce qu'**un composé de guanidine ayant au moins un groupe amino libre est mélangé avec du dextrane activé et glutamine-urée-lysine.
